# EUROPÄISCHE PATENTSCHRIFT

(11) **EP 4 277 595 B1**
(45) Veröffentlichungstag und Bekanntmachung des Hinweises auf die Patenterteilung: **12.03.2025**
(21) Anmeldenummer: 22700145.0
(22) Anmeldetag: 14.01.2022
(51) Int. Cl.: A61K 9/00

(54) **MEHRSCHICHTIGER ORALER DÜNNFILM**
MULTI-LAYER ORAL THIN FILM
FILM MINCE ORAL MULTICOUCHES

(30) Priorität: 15.01.2021 DE 102021100779
(43) Veröffentlichungstag der Anmeldung: 22.11.2023
(73) Patentinhaber: LTS Lohmann Therapie-Systeme AG, 56626 Andernach (DE)
(72) Erfinder: LINN, Michael, 55596 Waldböckelheim (DE); FICKER, Mario, 53179 Bonn (DE); NORELLI, Claudia, 56204 Hillscheid (DE)
(74) Vertreter: Held, Stephan
(86) Internationale Anmeldenummer: PCT/EP2022/050791
(87) Internationale Veröffentlichungsnummer: WO 2022/152877

(56) Entgegenhaltungen:
- MASEK JOSEF ET AL: "Multi-layered nanofibrous mucoadhesive films for buccal and sublingual administration of drug-delivery and vaccination nanoparticles - important step towards effective mucosal vaccines", JOURNAL OF CONTROLLED RELEASE, ELSEVIER, AMSTERDAM, NL, vol. 249, 25 July 2016 (2016-07-25), pages 183 - 195, XP029927819, ISSN: 0168-3659, DOI: 10.1016/J.JCONREL.2016.07.036
- PRAJAPATI VIPUL D. ET AL: "Pullulan based oral thin film formulation of zolmitriptan: Development and optimization using factorial design", INTERNATIONAL JOURNAL OF BIOLOGICAL MACROMOLECULES, vol. 107, 1 February 2018 (2018-02-01), NL, pages 2075 - 2085, XP055912413, ISSN: 0141-8130, Retrieved from the Internet <URL:https://www.sciencedirect.com/science/article/pii/S0141813017338114/pdfft?md5=8c7dd51a1721bdd1f7f264f7aa234d3a&pid=1-s2.0-S0141813017338114-main.pdf> DOI: 10.1016/j.ijbiomac.2017.10.082
- YOGYATA S PATHARE ET AL: "Polymers used for Fast Disintegrating Oral Films: A Review", INTERNATIONAL JOURNAL OF PHARMACEUTICAL SCIENCES REVIEW AND RESEARCH, 1 July 2013 (2013-07-01), pages 169 - 178, XP055566414, Retrieved from the Internet <URL:http://globalresearchonline.net/journalcontents/v21-1/29.pdf> [retrieved on 20190308]

## Beschreibung

Die vorliegende Erfindung betrifft einen mehrschichtigen oralen Dünnfilm, ein Verfahren zu dessen Herstellung, sowie den mehrschichtigen oralen Dünnfilm zur Verwendung als Arzneimittel.

Orale Dünnfilme sind dünne, mindestens einen pharmazeutisch aktiven Wirkstoff enthaltende Filme, die direkt in den Mundraum gelegt oder an die Mundschleimhaut angelegt werden und sich dort auflösen bzw. aufquellen und dabei den Wirkstoff abgeben. Dabei handelt es sich insbesondere um dünne, ein- oder mehrschichtige, wirkstoffhaltige Filme auf Polymerbasis, die, wenn sie auf die Schleimhaut, insbesondere die Mundschleimhaut, aufgebracht werden, den Wirkstoff direkt in diese abgeben können. Die sehr gute Durchblutung der Mundschleimhaut sorgt für einen schnellen Übergang des Wirkstoffs in den Blutkreislauf. Dieses Darreichungssystem hat den Vorteil, dass der Wirkstoff größtenteils durch die Schleimhaut resorbiert wird und damit der "First-Pass Metabolismus", der bei der konventionellen Darreichungsform eines Wirkstoffs in Tablettenform auftritt, vermieden wird. Der Wirkstoff kann in dem Film gelöst, emulgiert oder dispergiert werden.

Mašek Josef, et al. Multi-layered nanofibrous mucoadhesive films for buccal and sublingual administration of drug-delivery and vaccination nanoparticles - important step towards effective mucosal vaccines. J Control Release. 10. März 2017; Seiten 183-195, beschreibt Nanofaser-basierte mukoadhäsive Filme wurden für die oromukosale Verabreichung von Nanocarriern zur Verabreichung von Medikamenten und Impfstoffen entwickelt.

Prajapati Vipul et al. Pullulan based oral thin film formulation of zolmitriptan: Development and optimization using factorial design. Int J Biol Macromol., 1. Februar 2018; Seiten 2075-2085, beschreibt eine Studie zur Formulierung und Charakterisierung von Pullulan-basierten oralen Dünnfilmen (OTF) von Zolmitriptan mittels Lösungsmittel-Gießverfahren.

Yogyata S Pathare et al. "Polymers used for Fast Disintegrating Oral Films: A Review", International J. of Pharmaceutical Sciences Review and Research, 1. Juli 2013, Seiten 169-178, beschreibt die aktuellen Entwicklungen im Bereich der oralen Dünnschicht als neues Verabreichungssystem für die orale Verabreichung von Medikamenten.

Aus dem Stand der Technik bekannte orale Dünnfilme haben den Nachteil, dass sie, wenn sie längere Zeit an einer Stelle auf der Schleimhaut eines Patienten verbleiben sollen, einer dauerhaften Erosion ausgesetzt sind. Dies führt dazu, dass ein großer Teil des Materials abgeschluckt wird und so nicht die gewünschte Dauer am Applikationsort verweilen kann. Die Verweildauer kann aber durchaus für den transmucosalen Transport des pharmazeutisch aktiven Wirkstoffs von entscheidender Bedeutung sein. Des Weiteren haben viele Wirkstoffe einen schlechten Geschmack und werden als unangenehm empfunden, im speziellen bei Kontakt mit der Zunge.

Durch eine Schutzschicht auf der Rückseite kann verhindert werden, dass Flüssigkeit in die Formulierung eindringt und diese zu schnell auflöst, so dass der Wirkstoff maximale Zeit an der Applikationsstelle verweilen kann, um eine größtmögliche Permeation durch die Mucosa oder eine verzögerte Freisetzung zu erreichen. Ein weiterer Effekt der Rückschicht ist, dass sie verhindert, dass sich der verabreichte Film von dem Applikationsort löst und an anderer Stelle wie z.B. den Zähnen anhaftet. Ferner kann der durch den Wirkstoff hervorgerufenen schlechte Geschmack durch eine Rückschicht kaschiert werden.

Als Material für solche Rückschichten werden häufig unlösliche oder langsam lösliche Polymere bzw. Polymerfolien eingesetzt. Diese haben jedoch den Nachteil, dass sie nach Beendigung der Applikation entnommen oder abgeschluckt werden müssen.

Rückschichten aus langsam löslichen Polymeren haben zudem den Nachteil, dass sie auf langkettigen, hochmolekularen Polymeren basieren. Diese sind auf Grund ihrer hohen Viskosität schwer zu verarbeiten (lange Trocknungsdauer, unregelmäßige Filme). Des Weiteren neigen diese dazu, die Viskosität des Speichels im Mundraum zu erhöhen, wodurch ein schleimiges Gefühl entsteht.

Ein weiteres Problem stellt eine häufig unzureichende Verbindung zwischen den Teilen der wirkstoffhaltigen Schicht und der Rückschicht dar. Es besteht die Gefahr, dass sich beide Teile bei Lagerung voneinander lösen, wenn diese beispielsweise unterschiedlich auf Luftfeuchte reagieren.

Die Aufgabe der vorliegenden Erfindung besteht darin, vorstehend genannte Nachteile des Standes der Technik zu beheben. Insbesondere besteht die Aufgabe der vorliegenden Erfindung darin, einen mehrschichtigen oralen Dünnfilm bereitzustellen, der mindestens eine Rückschicht aufweist, wobei die Rückschicht insbesondere eine niedrige Schmelztemperatur, eine unbedenkliche Toxizität und eine glatte Oberfläche aufweisen soll. Ferner soll sich die Rückschicht möglichst langsam auflösen und so den applizierten Film vor Abschlucken und die Zunge vor direktem Kontakt mit dem Wirkstoff schützen, was einen unangenehmen Geschmack durch den Wirkstoff verhindert bzw. reduziert. Ferner soll der Wirkstoff vor zusätzlichem Speichel geschützt werden, was insbesondere von Vorteil ist, wenn der Wirkstoff oder die Wirkstoffpermeation pH-empfindlich ist und sich der pH-Wert durch zu viel Speichel ändern würde.

Ferner soll ein Herstellungsverfahren für einen solchen Film bereitgestellt werden, das eine feste Verbindung zwischen der wirkstoffhaltigen Schicht und der Rückschicht in dem oralen Dünnfilm gewährleistet.

Obige Aufgabe wird durch einen mehrschichtigen oralen Dünnfilm nach Anspruch 1 gelöst, insbesondere durch einen mehrschichtigen oraler Dünnfilm, umfassend eine mindestens ein Polymer und mindestens einen pharmazeutisch aktiven Wirkstoff enthaltende Matrixschicht und mindestens eine Rückschicht, wobei die mindestens eine Rückschicht mindestens ein Polyethylenglycol in einer Menge von 60 bis 100 Gew.-%, bezogen auf das Gesamtgewicht der mindestens einen Rückschicht, umfasst.

Ein solcher mehrschichtiger oraler Dünnfilm hat den Vorteil, dass sich die Schicht, umfassend ein Polyethylenglycol, aufgrund ihrer glatten Oberfläche, niedrigen Schmelztemperatur und unbedenklicher Toxizität gut als Rückschicht für orale Dünnfilme eignet. Die Schicht umfassend ein Polyethylenglycol löst sich langsam im Mund auf (langsamer als die Wirkstoffschicht) und schützt so den applizierten oralen Dünnfilm vor Abschlucken und zudem die Zunge vor direktem Kontakt mit dem Wirkstoff, was einen unangenehmen Geschmack durch den Wirkstoff verhindert bzw. reduziert. Des Weiteren wird der Wirkstoff vor zusätzlichem Speichel geschützt, was zum Beispiel von Vorteil ist, wenn der Wirkstoff oder die Wirkstoffpermeation pH-empfindlich ist und sich der pH-Wert durch zu viel Speichel ändern würde. Die unterschiedliche Rauigkeit der beiden Schichten (die Schicht umfassend ein Polyethylenglycol ist eher glatt und die wirkstoffhaltige Schicht ist eher sehr rau) kann vom Patienten ausgenutzt werden, um festzustellen, welche Seite des oralen Dünnfilms zur Mucosa hin appliziert wird. Aufgrund der unterschiedlichen Rauigkeit erkennt der Patient durch das Mundgefühl auch, ob der orale Dünnfilm richtig herum im Mund liegt.

Zusätzlich kann die Rückschicht mit Farbstoff eingefärbt werden, um eine bessere Sichtbarkeit zu erlangen.

Weiter ist es möglich Geschmacksstoffe einzuarbeiten, um das Mundgefühl während der Anwendung zu verbessern, was einen großen Vorteil gegenüber bekannten Folien darstellt.

In der vorliegenden Schrift kann "umfassend" auch "bestehend aus" bedeuten.

Unter Rückschicht wird eine Schicht des mehrschichtigen oralen Dünnfilms verstanden, die eine der äußersten Schichten des mehrschichtigen oralen Dünnfilms darstellt.

Polyethylenglycole (PEG) sind Verbindungen der allgemeinen Formel:

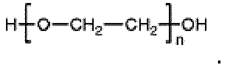

Höhermolekulare feste Polyethylenglycole (Schmelztemperatur etwa 65 °C) werden häufig auch als Polyethylenoxide oder auch Polyoxyethylene (Kurzzeichen PEO oder seltener PEOX) oder Polywachse bezeichnet. In der vorliegenden Schrift werde die Begriffe "Polyethylenglycol" und "Polyethylenoxid" gleichwertig verwendet.

Der erfindungsgemäße mehrschichtige orale Dünnfilm ist vorzugsweise dadurch gekennzeichnet, dass die Matrixschicht mindestens ein wasserlösliches Polymer umfasst.

Wasserlösliche Polymere umfassen chemisch sehr unterschiedliche, natürliche oder synthetische Polymere, deren gemeinsames Merkmal ihre Löslichkeit in Wasser oder wässrigen Medien ist. Voraussetzung ist, dass diese Polymere eine für die Wasserlöslichkeit ausreichende Anzahl an hydrophilen Gruppen besitzen und nicht vernetzt sind. Die hydrophilen Gruppen können nicht-ionisch, anionisch, kationisch und/oder zwitterionisch sein.

Unter wasserlöslich wird vorzugsweise eine Löslichkeit von größer als 100 g/L in Wasser bei 25°C verstanden.

Das mindestens eine wasserlösliche Polymer ist vorzugsweise ausgewählt aus der Gruppe bestehend aus Stärke und Stärkederivaten, Dextranen, Cellulosederivaten, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäuren, Polyacrylaten, Polyvinylpyrrolidonen, Vinvlpyrrolidon/Vinylacetat-Copolymeren, Polyvinylalkoholen, Polyethylenoxidpolymeren, Polyacrylamiden, Polyethylenglycolen, Gelatine, Collagen, Alginaten, Pektin, Pullulan, Traganth, Chitosan, Alginsäure, Arabinogalaktan, Galaktomannan, Agar, Agarose, Carrageen und natürlichen Gummen, wobei Polyvinylalkohole besonders bevorzugt sind.

Der erfindungsgemäße mehrschichtige orale Dünnfilm ist vorzugsweise dadurch gekennzeichnet, dass das mindestens eine Polymer, vorzugsweise das wasserlösliche Polymer, in einer Menge von 10 bis 90 Gew.-%, vorzugsweise von 20 bis 60 Gew.-%, besonders bevorzugt von 30 bis 50 Gew.-%, bezogen auf das Gesamtgewicht der wirkstoffhaltigen Matrixschicht, in der wirkstoffhaltigen Matrixschicht des mehrschichtigen oralen Dünnfilm vorhanden ist.

Der mindestens eine pharmazeutisch aktive Wirkstoff unterliegt prinzipiell keiner Beschränkung, ist aber vorzugsweise ausgewählt aus allen pharmazeutisch aktiven Wirkstoffen, die zur oralen und/oder transmucosalen Applikation geeignet sind.

Gemäß der vorliegenden Erfindung werden unter dem pharmazeutisch aktiven Wirkstoff auch alle pharmazeutisch akzeptablen Salze und Solvate des jeweiligen pharmazeutisch aktiven Wirkstoffs subsumiert.

Bevorzugt sind Wirkstoffe ausgewählt aus der Gruppe, umfassend die Wirkstoffklassen der Analgetika, Hormone, Hypnotika, Sedativa, Antiepileptika, Weckaminen, Psychoneurotropika, Neuro-Muskelblockern, Antispasmodika, Antihistaminika, Antiallergika, Cardiotonika, Antiarrhythmika, Diuretika, Hypotensiva, Vasopressoren, Antidepressiva, Antitussiva, Expectorantia, Thyroidhormonen, Sexualhormonen, Antidiabetika, Antitumor-Wirkstoffen, Antibiotika, Chemotherapeutika und Narcotika, wobei diese Gruppe nicht abschließend ist.

Besonders bevorzugt handelt es sich bei dem mindestens einen pharmazeutisch aktiven Wirkstoff um Ketamin und/oder ein pharmazeutisch akzeptables Salz oder Solvat davon, bevorzugt Ketamin·HCl.

Unter Ketamin wird (S)-(±)-2-(2-Chlorphenyl)-2-(methylamino)cyclohexan-1-on, (R)-(±)-2-(2-Chlorphenyl)-2-(methylamino)cyclohexan-1-on, sowie das Racemat (RS)-(±)-2-(2-Chlorphenyl)-2-(methylamino)cyclohexan-1-on verstanden.

Besonders bevorzugt liegt (S)-Ketamin oder ein pharmazeutisch akzeptables Salz davon, insbesondere (S)-Ketamin·HCl, als einziges Stereoisomer von Ketamin vor, da die analgetische und anästhetische Potenz von (S)-Ketamin etwa dreifach höher als die der (R)-Form ist.

Der Wirkstoffgehalt in der Matrixschicht kann innerhalb relativ weiten Grenzen variieren. Als geeignet kann ein Bereich von 10 bis 60 Gew.-%, bezogen auf das Trockengewicht der Matrixschicht, angegeben werden. In einer Ausführungsform liegt der Anteil an Wirkstoff in der Matrixschicht eher im unteren Bereich, z.B. wenn der Wirkstoff einen stark unangenehmen Geschmack aufweist, der mit einer größeren Menge an geschmacksmaskierenden Mitteln kompensiert werden muss. In diesem Fall kann als geeigneter Wirkstoffanteil ein Bereich von 10 bis 40 Gew.% angegeben werden. In einer anderen Ausführungsform liegt der Anteil an Wirkstoff in der erfindungsgemäßen Darreichungsform eher im oberen Bereich, wobei ein Gehalt von 40 bis 60 Gew.-% und insbesondere ein Gehalt von 45 bis 55 Gew.-% als besonders bevorzugt angegeben werden kann.

Besonders bevorzugt liegt (S)-Ketamin oder ein pharmazeutisch akzeptables Salz davon in einer Menge von 45 bis 55 Gew.-%, bezogen auf das Trockengewicht der wirkstoffhaltigen Matrixschicht, in der Matrixschicht vor.

Der erfindungsgemäße mehrschichtige orale Dünnfilm ist ferner vorzugsweise dadurch gekennzeichnet, dass die Matrixschicht mindestens einen Hilfsstoff ausgewählt aus der Gruppe, umfassend Farbstoffe, Aromastoffe, Süßstoffe, Weichmacher, geschmacksmaskierende Mittel, Emulgatoren, Enhancer, pH-Regulatoren, Feuchthaltemittel, Konservierungsmittel und/oder Antioxidationsmittel, umfasst.

Jeder dieser Hilfsstoffe ist vorzugsweise jeweils in einer Menge von 0,1 bis 40 Gew.-%, vorzugsweise von 0,1 bis 30 Gew.-%, besonders bevorzugt von 0,1 bis 15 Gew.-%, ganz besonders bevorzugt von 0,1 bis 10 Gew.-% oder 0,1 bis 5 Gew.-%, bezogen auf das Gesamtgewicht der Matrixschicht, in dieser Schicht enthalten.

Das mindestens eine Polyethylenglycol weist vorzugsweise ein mittleres Molekulargewicht von mindestens 20.000 g/mol bis 7.000.000 g/mol, vorzugsweise von 40.000 g/mol bis 500.000 g/mol, besonders bevorzugt von 95.000 g/mol bis 105.000 g/mol, insbesondere von etwa 100.000 g/mol auf.

Das Molekulargewicht wird aus den nachfolgend beschriebenen Rheologiemessungen abgeleitet.

Der erfindungsgemäße mehrschichtige orale Dünnfilm ist ferner vorzugsweise dadurch gekennzeichnet, dass das mindestens eine Polyethylenglycol eine Viskosität von 30 mPa s bis 50 mPa s, gemessen bei 25°C, aufweist.

Die angegebenen Viskositäten beziehen sich jeweils auf eine 5 Gew.-%-ige Lösung des Polyethylenglycols in Wasser und werden auf einem Brookfield Viskosimeter, Model RVF, mit Spindel No. 1 bei 50 rpm und bei einer Temperatur von 25°C gemessen.

Besonders bevorzugt wird ein Polyethylenglycol eingesetzt, das unter dem Handelsnamen POLYOX WSR N-10 (Dow Chemical) bekannt ist.

Der erfindungsgemäße mehrschichtige orale Dünnfilm ist ferner vorzugsweise dadurch gekennzeichnet, dass das mindestens eine Polyethylenglycol in einer Menge von 80 bis 100 Gew.-%, bezogen auf das Gesamtgewicht der mindestens einen Rückschicht, in der mindestens einen Rückschicht enthalten ist.

Der erfindungsgemäße mehrschichtige orale Dünnfilm ist ferner vorzugsweise dadurch gekennzeichnet, dass das mindestens eine Polyethylenglycol in einer Menge von 65 bis 100 Gew.-% oder 70 bis 100 Gew.-% oder 85 bis 100 Gew.-% oder 90 bis 100 Gew.-% oder 95 bis 100 Gew.-%, bezogen auf das Gesamtgewicht der mindestens einen Rückschicht, in der mindestens einen Rückschicht enthalten ist.

Der erfindungsgemäße mehrschichtige orale Dünnfilm ist ferner vorzugsweise dadurch gekennzeichnet, dass das mindestens eine Polyethylenglycol in einer Menge von 60 bis 97,5 Gew.-% oder 65 bis 97,5 Gew.-% oder 70 bis 97,5 Gew.% oder 80 bis 97, 5 Gew.-% oder 85 bis 97,5 Gew.-% oder 90 bis 97, 5 Gew.-% oder 95 bis 97,5 Gew.-%, bezogen auf das Gesamtgewicht der mindestens einen Rückschicht, in der mindestens einen Rückschicht enthalten ist.

Der erfindungsgemäße mehrschichtige orale Dünnfilm ist ferner vorzugsweise dadurch gekennzeichnet, dass das mindestens eine Polyethylenglycol in einer Menge von 60 bis 97,5 Gew.-% oder 65 bis 97,5 Gew.-% oder 70 bis 97,5 Gew.% oder 80 bis 97, 5 Gew.-% oder 85 bis 97,5 Gew.-% oder 90 bis 97, 5 Gew.-% oder 95 bis 97,5 Gew.-%, und zusätzlich 2 bis 2,5 Gew.-% mindestens eines Weichmachers, vorzugsweise Glycerol, bezogen auf das Gesamtgewicht der mindestens einen Rückschicht, in der mindestens einen Rückschicht enthalten sind.

Der erfindungsgemäße mehrschichtige orale Dünnfilm ist vorzugsweise dadurch gekennzeichnet, dass die mindestens eine Rückschicht mindestens einen Hilfsstoff ausgewählt aus der Gruppe, umfassend Farbstoffe, Aromastoffe, Süßstoffe, geschmacksmaskierende Mittel, Emulgatoren, Enhancer, pH-Regulatoren, Feuchthaltemittel, Konservierungsmittel und/oder Antioxidationsmittel, umfasst.

Jeder dieser Hilfsstoffe ist vorzugsweise jeweils in einer Menge von 0,1 bis 10 Gew.-%, vorzugsweise von 0,1 bis 5 Gew.-%, besonders bevorzugt von 0,1 bis 2,5 Gew.-%, bezogen auf das Gesamtgewicht der Rückschicht, in dieser Schicht enthalten.

Der erfindungsgemäße mehrschichtige orale Dünnfilm ist vorzugsweise dadurch gekennzeichnet, dass die mindestens eine Rückschicht mindestens einen Weichmacher, vorzugsweise Glycerol, vorzugsweise in einer Menge von 0,5 bis 5 Gew.-%, besonders bevorzugt in einer Menge von 2 bis 2,5 Gew.-%, bezogen auf das Gesamtgewicht der mindestens einen Rückschicht, enthält.

Der erfindungsgemäße mehrschichtige orale Dünnfilm ist prinzipiell in der Anzahl der enthaltenen Schichten nicht beschränkt.

So sind Ausführungsformen denkbar, bei denen der mehrschichtige orale Dünnfilm mehrere wirkstoffhaltige Matrixschichten aufweist.

Es sind auch Ausführungsformen denkbar, bei denen der mehrschichtige orale Dünnfilm zwei Rückschichten auf gegenüberliegenden Seiten des mehrschichtigen oralen Dünnfilms aufweist.

Insbesondere kann zwischen der mindestens einen Matrixschicht und der mindestens einen Rückschicht mindestens eine Klebeschicht vorhanden sein, um die mindestens eine Matrixschicht und die mindestens eine Rückschicht möglichst fest miteinander zu verbinden.

Unter Klebeschicht wird eine Schicht verstanden, die als Klebstoff, wie in der DIN EN 923:2016-03 definiert, wirken kann. Eine nicht-klebende Schicht kann demnach nicht als Klebstoff wie vorstehend definiert wirken.

Als Klebeschichten eignen sich insbesondere wasserlösliche Klebeschichten, wie diese in der DE 10 2014 127 452 A1 beschrieben werden, deren diesbezüglicher Inhalt hiermit vollständig einbezogen ist.

Geeignete Klebeschichten umfassen mindestens ein wasserlösliches Polymer und mindestens einen Weichmacher, wobei das mindestens eine wasserlösliche Polymer in der mindestens einen wasserlöslichen Klebeschicht Schellack, ein Vinylpyrrolidon-/Vinylacetat-Copolymer, ein Polyvinylcaprolactam-/Polyvinylacetat-/Polyethylenglycol-Copolymer, Hydroxypropylcellulose oder Hydroxypropylmethylcellulose und/oder Polyvinylpyrrolidon umfasst, und wobei der mindestens eine Weichmacher in der mindestens einen wasserlöslichen Klebeschicht Glycerin, Polyethylenglycol, insbesondere Polyethylenglycol 200, und/oder Tributylcitrat umfasst. Das Gewichtsverhältnis des mindestens einen wasserlöslichen Polymers zu dem mindestens einen Weichmacher in der mindestens einen Klebeschicht beträgt vorzugsweise etwa 85 bis 50 zu etwa 15 bis 50.

Eine derartige Klebeschicht, die mindestens ein wasserlösliches Polymer und mindestens einen Weichmacher enthält, kann als dazwischenliegende wasserlösliche Klebeschicht zwei weitere Schichten, die an sich nicht klebrig sind, fest miteinander verkleben und somit den Aufbau von mehrschichtigen oralen Filmen ohne mehrfacher Übereinanderbeschichtung ermöglichen

Der erfindungsgemäße mehrschichtige orale Dünnfilm ist vorzugsweise dadurch gekennzeichnet, dass der mehrschichtige orale Dünnfilm aus genau zwei Schichten, nämlich der mindestens ein Polymer und mindestens einen pharmazeutisch aktiven Wirkstoff enthaltenden Matrixschicht und einer mindestens ein Polyethylenglycol enthaltenden Rückschicht besteht.

Die wirkstoffhaltige Matrixschicht kann in einer Ausführungsform einen glatten Film darstellen.

Vorzugsweise ist der erfindungsgemäße mehrschichtige orale Dünnfilm dadurch gekennzeichnet, dass die Matrixschicht in Form eines Hohlräume aufweisenden verfestigten Schaums vorliegt.

Durch die Hohlräume und die damit verbundene größere Oberfläche der Filme wird insbesondere der Zutritt von Wasser bzw. Speichel oder anderen Körperflüssigkeiten in das Innere der Darreichungsform erleichtert und somit die Auflösung der Darreichungsform und die Wirkstofffreisetzung beschleunigt.

Bei einem schnell resorbierenden Wirkstoff kann zudem durch die schnelle Auflösung der Matrixschicht die transmukosale Resorption verbessert werden.

Zum anderen ist die Wandstärke der genannten Hohlräume vorzugsweise gering, da diese beispielsweise verfestigte Blasen darstellen, so dass eine schnelle Auflösung oder Zerstörung dieser Hohlräume stattfindet.

Ein weiterer Vorteil dieser Ausführungsform besteht darin, dass sich trotz des vergleichsweise hohen Flächengewichts durch die Konfektionierung als Schaum eine schnellere Trocknung realisieren lässt als bei einer vergleichbaren nicht geschäumten Zusammensetzung.

Vorzugsweise ist der erfindungsgemäße mehrschichtige orale Dünnfilm dadurch gekennzeichnet, dass die Hohlräume voneinander isoliert sind, und vorzugsweise in Gestalt von Blasen vorliegen, wobei die Hohlräume mit Luft oder einem Gas, vorzugsweise mit einem inerten Gas, besonders bevorzugt mit Stickstoff, Kohlendioxid, Helium oder einem Gemisch mindestens zweier dieser Gase, gefüllt sind.

Gemäß einer anderen Ausführungsform ist vorgesehen, dass die Hohlräume miteinander in Verbindung stehen, vorzugsweise indem sie ein zusammenhängendes, die Matrix durchdringendes Kanalsystem bilden.

Vorzugsweise haben die genannten Hohlräume einen Volumenanteil von 5 bis 98 %, bevorzugt von 50 bis 80 %, bezogen auf das Gesamtvolumen der Matrixschicht. Auf diese Weise wird der vorteilhafte Effekt, die Lösung der Matrixschicht zu beschleunigen, in günstiger Weise beeinflusst.

Ferner können dem Matrix-Polymer bzw. der Polymermatrix zur Schaumbildung oder dem erhaltenen Schaum vor oder nach dem Trocknen oberflächenaktive Stoffe bzw. Tenside hinzugefügt werden, um die Stabilität des Schaums vor oder nach dem Trocknen zu verbessern.

Ein weiterer Parameter, der die Eigenschaften der erfindungsgemäßen Darreichungsform beeinflusst, ist der Durchmesser der Hohlräume oder Blasen. Die Blasen oder Hohlräume werden vorzugsweise mit Hilfe einer Schaumaufschlagmaschine erzeugt, mit der der Durchmesser der Blasen in einem weiten Bereich, fast beliebig, eingestellt werden kann. So kann der mittlere Durchmesser, bestimmt zum Beispiel durch Mikrotomographie oder Mikroskopie der Blasen oder Hohlräume im Bereich von 1 bis 350 µm liegen. Besonders bevorzugt liegt der Durchmesser im Bereich von 40 und 200 µm.

Der erfindungsgemäße mehrschichtige orale Dünnfilm besitzt vorzugsweise eine raue Seite mit einer mittleren Rauheit Ra größer 2,0 µm und eine glatte Seite mit einer mittleren Rauheit Ra kleiner als 1,0 µm.

In einer anderen Ausführungsform ist der erfindungsgemäße mehrschichtige orale Dünnfilm dadurch gekennzeichnet, dass der mehrschichtige orale Dünnfilm eine raue Seite und eine glatte Seite besitzt, wobei die raue Seite eine mittleren Rauheit Ra von 1,0 µm mehr als die glatte Seite besitzt.

Die Messung der Rauheit kann dabei beispielsweise an einem KLA Tencor P15 Profilometer mit einer Messspitze von 2 µm Radius durchgeführt werden. Dazu wird beispielsweise auf beiden Seiten eines oralen Dünnfilms ein Oberflächenscan auf einer Fläche von 2 mm x 2 mm durchgeführt. Auf dem Scangebiet von 2 mm x 2 mm werden zufällig 3 Linienscans ausgewählt und die Rauheitsparameter Ra, Rq, Rp und Rv bestimmt.

Zur Bewertung der Probe können folgende Rauhigkeitsparameter bestimmt werden:
Ra: Mittlere Rauheit, gibt den mittleren Abstand eines Messpunktes zur Mittellinie an.
Rq: Quadratische Rauheit (auch RMS-Rauigkeit), wird aus dem Mittel der Abweichungsquadrate berechnet und entspricht dem quadratischen Mittel.
Rp: Peak-Höhe, Abstand zwischen der Mittellinie und dem größten Messwert.
Rv: Valley-Tiefe, Abstand zwischen der Mittellinie und dem kleinsten Messwert.

Der erfindungsgemäße mehrschichtige orale Dünnfilm ist ferner vorzugsweise dadruch gekennzeichnet, dass der mehrschichtige orale Dünnfilm eine mindestens 30% langsamere Freisetzung des mindestens einen pharmazeutisch aktiven Wirkstoffs aufweist wie die Matrixschicht alleine ohne Rückschicht.

Dies bedeutet, dass die Gegenwart der Rückschicht auf der Matrixschicht die Freisetzung des mindestens einen pharmazeutisch aktiven Wirkstoffs um mindestens 30% verzögern kann.

Der erfindungsgemäße orale Dünnfilm besitzt vorzugsweise eine Fläche von 0,5 cm² bis 10 cm², besonders bevorzugt von 1 cm² bis 8 cm².

Der erfindungsgemäße orale Dünnfilm ist vorzugsweise dadurch gekennzeichnet, dass das Flächengewicht des mehrschichtigen oralen Dünnfilms 10 bis 500 g/m², vorzugsweise 70 bis 400 g/m², beträgt.

Das Flächengewicht der Matrixschicht beträgt vorzugsweise mindestens 10 g/m², bevorzugter mindestens 20 g/m² oder mindestens 30 g/m² oder am meisten bevorzugt mindestens 50 g/m² oder ist kleiner als oder gleich 400 g/m², bevorzugter kleiner als oder gleich 350 g/m² oder kleiner als oder gleich 300 g/m² oder am meisten bevorzugt kleiner als oder gleich 150 g/m². Vorzugsweise beträgt das Flächengewicht 10 bis 400 g/m², bevorzugter 20 bis 350 g/m² oder 30 bis 300 g/m² und am meisten bevorzugt 50 bis 150 g/m².

Das Flächengewicht der Rückschicht ist wichtig für die Kontrolle des Auflösungsverhaltens und die Funktion der Rückschicht, um den Wirkstoff vor dem Auflösen im Speichel zu schützen. Eine bestimmte Dicke ist sinnvoll, um einen ausreichenden Schutz des Wirkstoffs sowie eine ausreichende Auflösungszeit zu gewährleisten, die normalerweise mindestens so lang sein sollte wie der aktive Permeationsbedarf und damit die Auflösungszeit der Matrixschicht.

Es ist daher bevorzugt, dass die Rückschicht ein Flächengewicht von mindestens 10 g/m², bevorzugter mindestens 20 g/m² oder mindestens 30 g/m², am meisten bevorzugt mindestens 50 g/m² oder ein Flächengewicht von kleiner als oder gleich 400 g/m², bevorzugter kleiner als oder gleich 350 g/m² oder kleiner als oder gleich 300 g/m² oder am meisten bevorzugt kleiner als oder gleich 150 g/m² oder ein Flächengewicht von 10 bis 400 g/m², bevorzugter von 20 bis 350 g/m² oder von 30 bis 300 g/m² am meisten bevorzugt von 50 bis 150 g/m² aufweist.

Bevorzugt weist die Matrixschicht und die mindestens eine Rückschicht jeweils eine Schichtdicke von etwa 10 µm bis etwa 500 µm, besonders bevorzugt von etwa 20 µm bis etwa 300 µm, auf.

Die Rückschicht kann hinsichtlich der Größe insbesondere gleich groß oder größer sein als die Matrixschicht. So sind in bestimmten Ausführungsformen die Größe der Rückschicht und die Größe der wirkstoffhaltigen Matrixschicht gleich, während in anderen Ausführungsformen die Rückschicht größer ist als die Oberfläche der Matrixschicht. Während eine Schichtstruktur mit gleicher Größe von Rück- und Matrixschicht einfacher herzustellen ist, da ein zweilagiges Blatt gestanzt werden kann, um die Schichtstruktur bereitzustellen, ist eine Schichtstruktur mit einer Rückschicht, die größer als die Matrixschicht ist, schwieriger herzustellen, bietet aber den Vorteil, dass die Gefahr des aktiven Auslaufens geringer ist, da auch der Rand der Matrixschicht von der Trägerschicht bedeckt wird.

Die vorliegende Erfindung betrifft ferner ein Verfahren zur Herstellung des erfindungsgemäßen mehrschichtigen oralen Dünnfilms umfassend die Schritte:
a) Herstellen und Ausstreichen einer Lösung oder Suspension, umfassend das mindestens eine Polyethylenglycol und anschließendes Trocknen der ausgestrichenen Lösung oder Suspension, um einen Film, umfassend das mindestens eine Polyethylenglycol zu erhalten,
b) Herstellen einer Lösung, Dispersion oder Schmelze, die mindestens das mindestens eine Polymer und den mindestens einen pharmazeutischen Wirkstoff umfasst,
b1) optional Aufschäumen der Lösung, Dispersion oder Schmelze aus Schritt b) durch Eintragen eines Gases oder Gasgemisches, durch chemische Gaserzeugung oder durch Entspannen eines gelösten Gases,
c) Ausstreichen der Lösung, Dispersion oder Schmelze aus Schritt b) bzw. der optional aufgeschäumten Lösung, Dispersion oder Schmelze aus Schritt b1) auf den in Schritt a) erhaltenen Film, umfassend das mindestens eine Polyethylenglycol, um einen Verbund zu erhalten,
d) Trocknen des in Schritt c) erhaltenen Verbundes, um einen mehrschichtigen oralen Dünnfilm zu erhalten.

Die Schritte a) und b) können in beliebiger Reihenfolge ausgeführt werden.

Dem Fachmann ist klar, dass der Schritt b1) nur dann notwendig ist, wenn die wirkstoffhaltige Matrixschicht in Form eines Hohlräume aufweisenden verfestigten Schaums vorliegen soll.

In Schritt c) können auch weitere Schichten, wie beispielsweise eine Klebeschicht, auf der Matrixschicht vorliegen.

Das Zusammenfügen der beiden Filme aus Schritt a) und b) kann prinzipiell durch dem Fachmann geläufige Verfahren erfolgen. So kann beispielsweise auf einen ersten Film mittels Beschichtung ein weiterer Film aufgebracht werden, wobei es prinzipiell unerheblich ist welcher Film auf welchen Film beschichtet wird, solange die Rückschicht eine der äußersten Schichten bildet. Ferner können die beiden Schichten durch eine Klebeschicht, wie vorstehend beschrieben, miteinander verbunden werden.

Vorzugsweise werden die Schichten mittels Wärmeeinwirkung miteinander verbunden. Dafür wird das Trocknen in Schritt d) bei einer Temperatur durchgeführt, die höher ist als die Schmelztemperatur der Matrixschicht und/oder der Rückschicht. Dies hat zur Folge, dass die Matrixschicht und/oder die Rückschicht etwa anschmilzt und so beim anschließenden Abkühlen für eine feste Verbindung der beiden Schichten sorgt.

Geeignete Temperaturen betragen 40 bis 100 °C, vorzugsweise 55 bis 80 °C. Vorzugsweise wird für eine Dauer von 10 bis 60 min getrocknet.

In einer weiteren Ausführungsform ist der erfindungsgemäße Verfahren dadurch gekennzeichnet, dass die vorstehend genannten Schritte c) und d) durch die Schritte
c2) Ausstreichen der Lösung, Dispersion oder Schmelze aus Schritt b) bzw. der optional aufgeschäumten Lösung, Dispersion oder Schmelze aus Schritt b1), um einen Film, umfassend das mindestens eine Polymer und den mindestens einen pharmazeutischen Wirkstoff, zu erhalten und
d2) Verbinden der in Schritt a) und c2) erhaltenen Filme, vorzugsweise durch Laminieren mittels Wärmeeinwirkung über den Schmelzpunkt eines der in den Filmen enthaltenen Polymere, um einen mehrschichtigen oralen Dünnfilm zu erhalten,
ersetzt werden.

Durch das modifizierte Verfahren sind auch Ausführungsformen des mehrschichtigen oralen Dünnfilms, wobei beide Seiten der wirkstoffhaltigen Matrixschicht von einer Schicht, umfassend mindestens ein Polyethylenglycol, bedeckt sind, einfach herstellbar. Solche mehrschichtigen oralen Dünnfilme lösen sich sehr langsam.

Die vorliegende Erfindung betrifft ferner einen mehrschichtigen oralen Dünnfilm erhältlich nach dem vorstehend beschriebenen Verfahren.

Außerdem betrifft die vorliegende Erfindung einen mehrschichtigen oralen Dünnfilm, wie vorstehend beschrieben, oder erhältlich nach dem vorstehend beschriebenen Verfahren als Arzneimittel.

Die vorliegende Erfindung betrifft zudem einen mehrschichtigen oralen Dünnfilm, wie vorstehend beschrieben oder erhältlich nach dem vorstehend beschriebenen Verfahren, wobei Ketamin, vorzugsweise S-Ketamin, als pharmazeutisch aktiver Wirkstoff eingesetzt wird, zur Verwendung in der Behandlung von Schmerzen und/oder Depressionen, insbesondere zur Reduzierung des Suizidrisikos, und/oder zur Verwendung als Allgemeinanästhetikum, vorzugsweise zur Einleitung und Durchführung einer Vollnarkose oder als Ergänzung bei Regionalanästhesien und/oder als Analgetikum.

Die vorstehend für den erfindungsgemäßen mehrschichtigen oralen Dünnfilm aufgeführten bevorzugten Ausführungsformen gelten auch für das erfindungsgemäße Verfahren, den durch dieses Verfahren erhaltenen mehrschichtigen oralen Dünnfilm und dessen Verwendung als Arzneimittel.

Die Erfindung wird nachfolgend anhand von nicht beschränkenden Beispielen näher erläutert.

### Beispiel 1:

Beispielformulierung aus einer Polyethylenglycol (Polyox) Rückschicht und einer S-Ketamin haltigen Matrixschicht in Form eines Schaums.

**Tabelle 1:**

| Material | Menge [Gew.-%] |
|---|---|
| **Wirkstoffhaltige Matrixschicht** | |
| S-Ketamin·HCl | 50,00 |
| PVA 4-88 | 41,70 |
| Saccharin Na | 1,00 |
| Sucralose | 2,00 |
| Glycerol | 2,30 |
| Geschmacksstoff 1 | 1,00 |
| Geschmacksstoff 2 | 2,00 |
| Prozesslösungsmittel | Wasser |

| **Rückschicht** | |
|---|---|
| Polyox WSR N10 | 97,50 |
| Glycerol | 2,30 |
| Farbstoff | 0,20 |
| Prozesslösungsmittel | Wasser |
| Flächengewicht (trocken) | Wirkstoffhaltige Matrixschicht: |
| | 118,7 g/m² |
| | Rückschicht: |
| | 100 g/m² |
| Beschichtungsgewicht (inkl. Restwasser) | Wirkstoffhaltige Matrixschicht: |
| | 121,9 g/m² |
| | Rückschicht: |
| | 101,5 g/m² |
| S-Ketamin Base in mg/oralem Dünnfilm | 14,9 mg / 2,9 cm² |
| | Entspricht 17,2 mg S-Ketamin·HCl |

| | |
|---|---|
| PVA 4-88: Polyvinylakohol Polyox WSR N10: Polyethylenglycol mit einem MW von 100,000 g/mol von Dow Chemical | |

Der mehrschichtige orale Dünnfilm gemäß der obigen Zusammensetzung wurde wie folgt hergestellt.

Die Rohstoffe der Rückschicht werden durch bekannte Wäge-und Rührtechniken sowie mit geeigneten Rührwerkzeugen und Motoren zu einer homogenen Masse verarbeitet. Diese wird dann mit Hilfe von Beschichtungswerkzeugen (z.B. Filmziehgeräte) auf einen bahnförmigen Träger beschichtet (mit einem bestimmten Flächengewicht). Der feuchte Film wird mit Hilfe eines Trockenschrankes bei 70°C für 15 min getrocknet.

Die Rohstoffe der wirkstoffhaltigen Matrixschicht werden ebenfalls durch bekannte Wäge-und Rührtechniken sowie mit geeigneten Rührwerkzeugen und Motoren zu einer homogenen Masse verarbeitet. Diese Masse wird wiederum mit Techniken des Rührens aufgeschäumt und der somit erhaltene Schaum wird auf die trockene Rückschicht beschichtet. Dies geschieht wieder mit Hilfe von Beschichtungswerkzeugen (z.B. Filmziehgeräte). Der erhaltene Film, bestehend aus Rückschicht und Matrixschicht, wird mit Hilfe eines Trockenschranks noch einmal bei 70°C für 15 min getrocknet.

Dieser mehrschichtige Film kann nach dem Trocknen in kleinere orale Dünnfilme konfektioniert werden.

### Beispiel 2:

Beispielformulierung aus einer Polyethylenglycol (Polyox) Rückschicht und einer S-Ketamin haltigen Matrixschicht in Form eines Schaums, wobei die Schichten einzeln hergestellt und schließend durch Laminieren miteinander verbunden werden.

**Tabelle 2:**

| **Wirkstoffhaltige Matrixschicht** | **Menge [Gew.-%]** |
|---|---|
| (S)-Ketamin HCl | 50,00 % |
| PVA 4-88 | 41,70 % |
| Saccharin Na | 1,00 % |
| Sucralose | 2,00 % |
| Glycerol | 2,30 % |
| Geschmacksstoff 1 | 1,00 % |
| Geschmacksstoff 2 | 2,00 % |
| Prozesslösungsmittel | Aqua purificata |
| Flächengewicht (tocken) | 117,3 g/m² |
| Flächengewicht (incl. res. Lösungsmittel) | 121,9 g/m² |
| | |

| **Rückschicht** | **Menge [Gew.-%]** |
|---|---|
| Polyox WSR N10 | 97,50 % |
| Glycerol | 2,3 % |
| Farbstoff | 0,20 % |
| Prozesslösungsmittel | Aqua purificata |
| Flächengewicht (trocken) | 98,2 g/m² |
| Flächengewicht (incl. res. Lösungsmittel) | 101,5 g/m² |

Die Rohstoffe der Rückschicht werden durch bekannte Wäge-und Rührtechniken sowie mit geeigneten Rührwerkzeugen und Motoren zu einer homogenen Masse verarbeitet. Diese wird dann mit Hilfe von Beschichtungswerkzeugen (z.B. Filmziehgeräte) auf einen bahnförmigen Träger beschichtet (mit einem bestimmten Flächengewicht). Der feuchte Film wird mit Hilfe eines Trockenschrankes bei 70°C für 15 min getrocknet.

Die Rohstoffe der wirkstoffhaltigen Matrixschicht werden ebenfalls durch bekannte Wäge-und Rührtechniken sowie mit geeigneten Rührwerkzeugen und Motoren zu einer homogenen Masse verarbeitet. Diese Masse wird wiederum mit Techniken des Rührens aufgeschäumt und der somit erhaltene Schaum wird auf einen bahnförmigen Träger beschichtet. Dies geschieht wieder mit Hilfe von Beschichtungswerkzeugen (z.B. Filmziehgeräte). Der feuchte Film wird mit Hilfe eines Trockenschrankes bei 70°C für 15 min Zeit getrocknet.

Die beiden Schichten wurden durch Laminieren zusammengefügt. Hierzu wurde die Rückschicht kurzeitig auf 70 °C erwärmt und anschließend mit der wirkstoffhaltigen Matrixschicht durch Laminieren verbunden.

Dieser mehrschichtige Film kann in kleinere orale Dünnfilme konfektioniert werden.

Bei diesem mehrschichtigen oralen Dünnfilm dient eine wirkstofffreie Polyoxschicht als Rückschicht für die eigentliche wirkstoffhaltige Matrixschicht des oralen Dünnfilms. Die Schutzschicht kann den Geschmack des Wirkstoffs maskieren und ein schnelles Abschlucken des Wirkstoffes vermeiden und somit die oro-mukosale Bioverfügbarkeit erhöhen. Im Speziellen zeichnet sich der erfindnungsgemäße orale Dünnfilm dadurch aus, dass der Wirkstoffstrich bei der Herstellung direkt auf die Polyoxschicht beschichtet werden kann.

Durch Trocknung oberhalb des Schmelzpunktes von Polyox verbinden sich wirkstoffhaltige Matrixschicht und die Rückschicht zu einem festen Verbund. Eine Klebeschicht ist somit nicht notwendig.

Des Weiteren kann der Patient an der unterschiedlichen Rauigkeit der Schichten (die Polyox enthaltende Rückschicht ist sehr glatt bzw. folienähnlich und die wirkstoffhaltige Matrixschicht in Form eines Schaums sehr rau) erkennen, welche Seite vom OTF auf die Mucosa aufgelegt werden soll und ob die richtige Seite auch so appliziert wurde (unterschiedliches Mundgefühl je nachdem welche Schicht oben bzw. unten liegt).

### Eigenschaften Zerfallszeit:

Die Zerfallszeit eines mehrschichtigen oralen Dünnfilms (mit Rückschicht) mit einer Zusammensetzung gemäß Tabelle 2 wurde mit der Zerfallszeit eines einschichtigen oralen Dünnfilms mit einer Zusammensetzung gemäß Tabelle 3 (ohne Rückschicht) verglichen. Die Herstellung erfolgte analog zudem vorstehend beschriebenen Verfahren für den oralen Dünnfilms (mit Rückschicht) mit einer Zusammensetzung gemäß Tabelle 2.

**Tabelle 3:**

| **Wirkstoffhaltige Matrixschicht** | **Menge [Gew.-%]** |
|---|---|
| (S)-Ketamin HCl | 50,00 % |
| PVA 4-88 | 41,70 % |
| Saccharin Na | 1,00 % |
| Sucralose | 2,00 % |
| Glycerol | 2,30 % |
| Geschmacksstoff 1 | 1,00 % |
| Geschmacksstoff 2 | 2,00 % |
| Prozesslösungsmittel | Aqua purificata |
| Flächengewicht (trocken) | 117,3 g/m² |
| Flächengewicht (incl. res. Lösungsmittel) | 121,9 g/m² |

Die Zerfallszeiten der hergestellten oralen Dünnfilme wurden in Anlehnung an USP 701 mit einem Tablettenzerkleinerungsgerät (Pharma-Test DIST-3 Triple Basket Tablet Disintegration Tester, 30 Hübe pro Minute über eine Distanz von 55 mm, in 1l pH 6,8 Phosphatpuffer) gemessen. Orale Dünnfilme mit einer Größe von 2,72 cm² wurden in einen Korb ("Sinker") gelegt und in einem am Instrument befestigten Glasrohr positioniert. Schließlich wurde die Zeit bestimmt, bis sich nur noch Reste der oralen Dünnfilme im Korb befanden.

**Tabelle 4:**

| Oraler Dünnfilm | Zerfallszeit |
|---|---|
| nur wirkstoffhaltige Matrixschicht (Tabelle 3) | 3 s |
| wirkstoffhaltige Matrixschicht mit Rückschicht (Tabelle 2) | 44 s |

### Wirkstofffreisetzung:

Es wurde ferner die in vitro Freisetzung des Wirkstoffs aus den oralen Dünnfilmen mit den Zusammensetzungen gemäß Tabelle 2 und 3 wie folgt ermittelt:

### Freisetzungsmethode:

Bei der in-vitro Freisetzung wird S-Ketamine aus S-Ketamine HCl oralen Dünnfilmen (OTF) freigesetzt und bestimmt. Der Wirkstoff wird in Phosphatpuffer pH 6.8 USP freigesetzt und dann durch eine Gradienten-Umkehrphasen HPLC-Methode bestimmt. Die Quantifizierung erfolgte gegen einen externen Standard.

Die Freisetzung wird mit Dissolution Apparatus 2 - (Paddle over sinker) gemäß USP <711> durchgeführt.

| | |
|---|---|
| Sinker: | Stainless Steel Capsule Sinker with 10 Spirals, 31.0 x 11.0 mm Capacity (Sotax Style) |
| Rührgeschwindigkeit: | 50 rpm |
| Abstand zwischen dem Vesselboden und der Unterkante des Paddle: | 25 mm ± 2 mm |
| Temperatur: | 37°C ± 0.5°C |
| Freisetzungsmedium: | Phosphatpuffer pH 6.8 USP |
| Volumen Freisetzungsmedium: | 900 mL |
| Probennahmezeitpunkte: | 1, 3,5 10 und 15 min |
| Probenvolumen: | 5,0 mL |

**Tabelle 5:**

| In-vitro Freisetzung [% von LC*] | | nur wirkstoffhaltige Matrixschicht (Tabelle 3) | wirkstoffhaltige Matrixschicht mit Rückschicht (Tabelle 2) |
|---|---|---|---|
| nach 1min | Mean | 101 | 60 |
| | Min | 96 | 47 |
| | Max | 106 | 75 |
| | RSD [%] | 4,2 | 19,9 |
| nach 3min | Mean | 103 | 102 |
| | Min | 100 | 97 |
| | Max | 108 | 111 |
| | RSD [%] | 3,6 | 4,7 |
| nach 5min | Mean | 104 | 104 |
| | Min | 100 | 100 |
| | Max | 109 | 105 |
| | RSD [%] | 4,0 | 1,9 |
| nach 10min | Mean | 104 | 103 |
| | Min | 100 | 101 |
| | Max | 109 | 105 |
| | RSD [%] | 3,9 | 1,4 |
| nach 15min | Mean | 103 | 104 |
| | Min | 100 | 101 |
| | Max | 108 | 106 |
| | RSD [%] | 3,9 | 1,9 |

| | | | |
|---|---|---|---|
| *Label Claim = Sollgehalt | | | |

### Beispiel 3:

Ein mehrschichtiger oraler Dünnfilm mit einer Zusammensetzung gemäß Tabelle 6, wobei eine wirkstoffhaltige Matrixschicht von beiden Seiten mit einer Rückschicht bedeckt ist, wurde hergestellt.

**Tabelle 6:**

| Material | Menge [Gew.-%] |
|---|---|
| **Wirkstoffhaltige Matrixschicht** | |
| S-Ketamin·HCl | 50,00 |
| PVA 4-88 | 41,70 |
| Saccharin Na | 1,00 |
| Sucralose | 2,00 |
| Glycerol | 2,30 |
| Geschmacksstoff 1 | 1,00 |
| Geschmacksstoff 2 | 2,00 |
| Prozesslösungsmittel | Wasser |

| **2 x Rückschicht (jeweils)** | |
|---|---|
| Polyox WSR N10 | 97,50 |
| Glycerol | 2,30 |
| Farbstoff | 0,20 |
| Prozesslösungsmittel | Wasser |
| Flächengewicht (trocken) | Wirkstoffhaltige Matrixschicht: |
| | 118,7 g/m² |
| | Rückschicht: |
| | 100 g/m² |
| Beschichtungsgewicht (inkl. Restwasser) | Wirkstoffhaltige Matrixschicht: |
| | 121,9 g/m² |
| | Rückschicht: |
| | 101,5 g/m² |
| S-Ketamin Base in mg/oralem Dünnfilm | 14 mg / 2,72 cm² |
| | Entspricht 16,1 mg S-Ketamin·HCl |

Die Rohstoffe der beiden Rückschichten werden durch bekannte Wäge-und Rührtechniken sowie mit geeigneten Rührwerkzeugen und Motoren zu einer homogenen Masse verarbeitet. Diese wird dann mit Hilfe von Beschichtungswerkzeugen (z.B. Filmziehgeräte) auf einen bahnförmigen Träger beschichtet (mit einem bestimmten Flächengewicht). Der feuchte Film wird mit Hilfe eines Trockenschrankes bei 70°C für 15 min getrocknet.

Die Rohstoffe der wirkstoffhaltigen Matrixschicht werden ebenfalls durch bekannte Wäge-und Rührtechniken sowie mit geeigneten Rührwerkzeugen und Motoren zu einer homogenen Masse verarbeitet. Diese Masse wird wiederum mit Techniken des Rührens aufgeschäumt und der somit erhaltene Schaum wird auf einen bahnförmigen Träger beschichtet. Dies geschieht wieder mit Hilfe von Beschichtungswerkzeugen (z.B. Filmziehgeräte). Der feuchte Film wird mit Hilfe von einem Trockenschrank bei 70°C für 15 min getrocknet.

Die beiden Rückschichten und die dazwischenliegende Wirkstoffschicht wurden durch Laminieren zusammengefügt. Hierzu wurde eine Rückschicht kurzeitig auf 70 °C erwärmt und anschließend mit der wirkstoffhaltigen Matrixschicht durch Laminieren verbunden. Darauf wurde eine zweite Rückschicht kurzeitig auf 70 °C erwärmt und anschließend auf die freie Seite der wirkstoffhaltigen Matrixschicht des vorherigen Laminates laminiert. Es wurde somit ein Laminat der Zusammensetzung Rückschicht - wirkstoffhaltige Matrixschicht - Rückschicht erhalten.

Das erhaltene Laminat kann in kleinere orale Dünnfilme konfektioniert werden.

### Beispiel 4

Ein mehrschichtiger oraler Dünnfilm mit einer Zusammensetzung gemäß Tabelle 7 wurde analog zu Beispiel 2 hergestellt. Die wirkstoffhaltige Matrixschicht wurde dabei nicht geschäumt.

**Tabelle 7:**

| **Rückschicht** | **Menge [Gew.-%]** |
|---|---|
| Polyox WSR N10 | 97,50 % |
| Glycerol | 2,3 % |
| Farbstoff | 0,20 % |
| Prozesslösungsmittel | Aqua purificata |
| | |
| Flächengewicht (trocken) | 98,2 g/m² |
| Flächengewicht (incl. res. Prozesslösungsmittel ) | 101,5 g/m² |
| | |

| **Wirkstoffhaltige Matrixschicht** | **Menge [Gew.-%]** |
|---|---|
| HPMC 2910 (603) | 39,5 |
| HPMC 2910 (60SH50) | 10,0 |
| S-Ketamin | 41,0 |
| Saccharin Na | 2,0 |
| Sucralose | 1,0 |
| Glycerol | 3,5 |
| Geschmacksstoff | 3,0 |
| Prozesslösungsmittel | Aqua purificata |
| Flächengewicht (trocken) | 175 g/m² |
| Flächengewicht (incl. res. Prozesslösungsmittel ) | 184,5 g/m² |
| Bemerkung | Nicht geschäumt |

| | |
|---|---|
| HPMC 2910 (603): Hydroxypropylmethylcellulose 2910 (603) HPMC 2910 (60SH50): Hydroxypropylmethylcellulose 2910 (60SH50) Die eingesetzten HPMCs unterscheiden sich in der Viskosität einer 2% wässrigen Lösung: 603: 3 mPaS vs. 60SH50: 50 mPaS | |

Die zwei Schichten wurden separat hergestellt und durch Erhitzen auf 70 °C durch Laminieren miteinander verbunden. Nach dem Abkühlen waren die beiden Schichten fest verbunden.

### Beispiel 5

Eine morphologische Oberflächenuntersuchung wurde an einem KLA Tencor P15 Profilometer mit einer Messspitze von 2 µm Radius durchgeführt. Auf beiden Seiten eines OTF von Beispiel 1 wurde ein Oberflächenscan auf einer Fläche von 2 mm x 2 mm durchhgeführt. Auf dem Scangebiet von 2 mm x 2 mm wurden zufällig 3 Linienscans ausgewählt und die Rauheitsparameter Ra, Rq, Rp und Rv bestimmt.

Zur Bewertung der Probe wurden folgende Rauhigkeitsparameter bestimmt:
Ra: Mittlere Rauheit, gibt den mittleren Abstand eines Messpunktes zur Mittellinie an.
Rq: Quadratische Rauheit (auch RMS-Rauigkeit), wird aus dem Mittel der Abweichungsquadrate berechnet und entspricht dem quadratischen Mittel.
Rp: Peak-Höhe, Abstand zwischen der Mittellinie und dem größten Messwert.
Rv: Valley-Tiefe, Abstand zwischen der Mittellinie und dem kleinsten Messwert.

Die Rauhigkeitsparameter zeigen, dass der OTF zwei verschieden raue Seiten hat. Die Seite der Rückschicht ist sehr glatt, vergleichbar mit glatten Materialien wie poliertem Stahl. Die Seite der matrixhaltigen Wirkstoffschicht ist dagegen deutlich rauer. Durch die unterschiedliche Rauigkeit der beiden OTF Seiten kann ein Patient durch Fühlen erkennen, welche Seite die Rückschicht ist und welche Seite die wirkstoffhaltige Seite ist. Hierdurch kann zum Beispiel die richtige Lage des OTF im Mund erkannt werden (z.B. raue Seite nach unten zur Mundschleimhaut hin, glatte Rückschicht nach oben).

### OTF auf Seite der Rückschicht:

| Messung | Ra/µm | Rq/µm | Rp/µm | Rv/µm |
|---|---|---|---|---|
| 1 | 0,76 | 1,06 | 4,85 | 1,40 |
| 2 | 0,55 | 0,86 | 5,54 | 0,87 |
| 3 | 0,74 | 1,15 | 6,64 | 2,09 |
| Mittelwert | 0,68 | 1,02 | 5,68 | 1,45 |

### OTF auf Seite der wirkstoffhaltigen Matrixschicht :

| Messung | Ra/µm | Rq/µm | Rp/µm | Rv/µm |
|---|---|---|---|---|
| 1 | 3,17 | 3,89 | 11,64 | 11,15 |
| 2 | 2,73 | 3,84 | 11,59 | 19,96 |
| 3 | 3,84 | 4,74 | 10,61 | 12,07 |
| Mittelwert | 3,24 | 4,16 | 11,28 | 14,39 |

### Beispiel 6:

Es wurden weitere Freisetzungsmessungen mit Proffildissolution und einer höheren zeitlichen Auflösung als in Tabelle 5 durchgeführt.

Verglichen wurden:
- wirkstoffhaltige Matrixschicht (Tabelle 3)
- wirkstoffhaltige Matrixschicht mit Rückschicht auf einer Seite (Tabelle 2)
- wirkstoffhaltige Matrixschicht mit Rückschicht auf beiden Seiten (Tabelle 7)

Dazu wird S-Ketamine aus S-Ketamine HCl oralen Dünnfilmen (OTF) freigesetzt und bestimmt. Der Wirkstoff wird in Phosphatpuffer pH 6.8 USP freigesetzt und dann durch eine Gradienten-Umkehrphasen HPLC-Methode bestimmt. Die Quantifizierung erfolgte gegen einen externen Standard.

Die Freisetzung wird mit Dissolution Apparatus 2 - (Paddle over sinker) gemäß USP <711> durchgeführt.

| | |
|---|---|
| Sinker: | Stainless Steel Capsule Sinker with 10 Spirals, 31.0 x 11.0 mm Capacity (Sotax Style) |
| Rührgeschwindigkeit: | 50 rpm |
| Abstand zwischen dem Vesselboden und der Unterkante des Paddle: | 25 mm ± 2 mm |
| Temperatur: | 37°C ± 0.5°C |
| Freisetzungsmedium: | Phosphatpuffer pH 6,8 USP |
| Volumen Freisetzungsmedium: | 900 mL |
| Probennahmezeitpunkte: | 1, 3,5 10 und 15 min |
| Probenvolumen: | 5,0 mL |

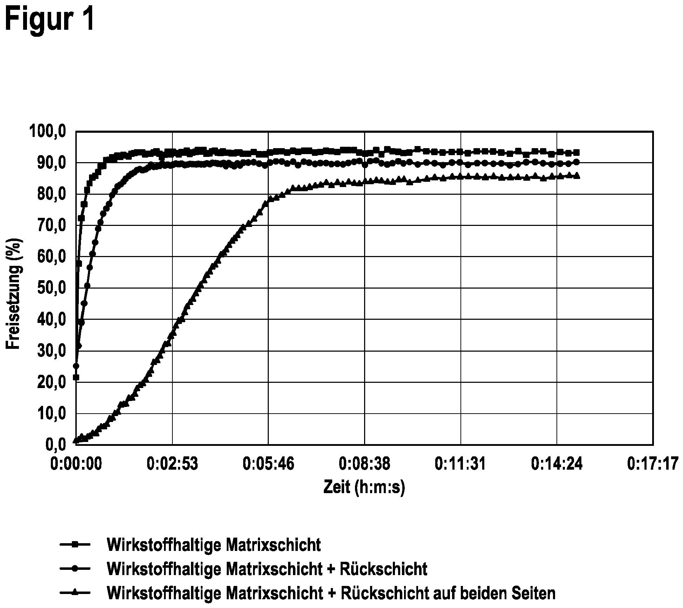

Die Ergebnisse sind in Figur 1 dargestellt. Der Fachmann kann erkennen, dass sich mit der Rückschicht das Freisetzungsprofil der Matrixschicht variieren lässt.

## Patentansprüche

1. Mehrschichtiger oraler Dünnfilm, umfassend eine mindestens ein Polymer und mindestens einen pharmazeutisch aktiven Wirkstoff enthaltende Matrixschicht und mindestens eine Rückschicht, wobei die mindestens eine Rückschicht mindestens ein Polyethylenglycol in einer Menge von 60 bis 100 Gew.-%, bezogen auf das Gesamtgewicht der mindestens einen Rückschicht, umfasst.

2. Mehrschichtiger oraler Dünnfilm gemäß Anspruch 1, wobei die Matrixschicht mindestens ein wasserlösliches Polymer umfasst.

3. Mehrschichtiger oraler Dünnfilm gemäß Anspruch 2, wobei das mindestens eine wasserlösliche Polymer ausgewählt ist aus der Gruppe, umfassend Stärke und Stärkederivate, Dextrane, Cellulosederivate, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäure, Polyacrylate, Polyvinylpyrrolidone, Vinvlpyrrolidon/Vinylacetat-Copolymere, Polyvinylalkohole, Polyethylenoxidpolymere, Polyacrylamide, Polyethylenglycole, Gelatine, Collagen, Alginaten, Pektin, Pullulan, Traganth, Chitosan, Alginsäure, Arabinogalaktan, Galaktomannan, Agar, Agarose, Carrageen und natürlichen Gummen.

4. Mehrschichtiger oraler Dünnfilm gemäß irgendeinem der vorhergehenden Ansprüche, wobei der mindestens eine pharmazeutisch aktive Wirkstoff ausgewählt ist aus der Gruppe, umfassend die Wirkstoffklassen der Analgetika, Hormone, Hypnotika, Sedativa, Antiepileptika, Weckaminen, Psychoneurotropika, Neuro-Muskelblockern, Antispasmodika, Antihistaminika, Antiallergika, Cardiotonika, Antiarrhythmika, Diuretika, Hypotensiva, Vasopressoren, Antidepressiva, Antitussiva, Expectorantia, Thyroidhormonen, Sexualhormonen, Antidiabetika, Antitumor-Wirkstoffen, Antibiotika, Chemotherapeutika und Narcotika, wobei der mindestens eine pharmazeutisch aktive Wirkstoff vorzugsweise Ketamin, besonders bevorzugt (S)-Ketamin, umfasst.

5. Mehrschichtiger oraler Dünnfilm gemäß irgendeinem der vorhergehenden Ansprüche, wobei die Matrixschicht ferner mindestens einen Hilfsstoff ausgewählt aus der Gruppe, umfassend Farbstoffe, Aromastoffe, Süßstoffe, Weichmacher, geschmacksmaskierende Mittel, Emulgatoren, Enhancer, pH-Regulatoren, Feuchthaltemittel, Konservierungsmittel und/oder Antioxidationsmittel, umfasst.

6. Mehrschichtiger oraler Dünnfilm gemäß irgendeinem der vorhergehenden Ansprüche, wobei das mindestens eine Polyethylenglycol ein mittleres Molekulargewicht von 20000 g/mol bis 7000000 g/mol, vorzugsweise von 40000 g/mol bis 500000 g/mol, besonders bevorzugt von 95.000 g/mol bis 105.000 g/mol, insbesondere von etwa 100.000 g/mol, aufweist.

7. Mehrschichtiger oraler Dünnfilm gemäß irgendeinem der vorhergehenden Ansprüche, wobei das mindestens eine Polyethylenglycol eine Viskosität von 30 mPa s bis 50 mPa s, gemessen in 5 Gew.-%-iger wässriger Lösung bei 25°C, aufweist.

8. Mehrschichtiger oraler Dünnfilm gemäß irgendeinem der vorhergehenden Ansprüche, wobei das mindestens eine Polyethylenglycol in einer Menge von 80 bis 100 Gew.-%, bezogen auf das Gesamtgewicht der mindestens einen Rückschicht, in der mindestens einen Rückschicht enthalten ist.

9. Mehrschichtiger oraler Dünnfilm gemäß irgendeinem der vorhergehenden Ansprüche, wobei die mindestens eine Rückschicht mindestens einen Weichmacher, vorzugsweise Glycerol, vorzugsweise in einer Menge von 0,5 bis 5 Gew.-%, bezogen auf das Gesamtgewicht der mindestens einen Rückschicht, enthält.

10. Mehrschichtiger oraler Dünnfilm gemäß irgendeinem der vorhergehenden Ansprüche, wobei der mehrschichtige orale Dünnfilm aus genau zwei Schichten, nämlich der mindestens ein Polymer und mindestens einen pharmazeutisch aktiven Wirkstoff enthaltenden Matrixschicht und der mindestens ein Polyethylenglycol enthaltenden Rückschicht besteht.

11. Mehrschichtiger oraler Dünnfilm gemäß irgendeinem der vorhergehenden Ansprüche, wobei die Matrixschicht in Form eines Hohlräume aufweisenden verfestigten Schaums vorliegt.

12. Mehrschichtiger oraler Dünnfilm gemäß Anspruch 11, wobei die Hohlräume voneinander isoliert sind und vorzugsweise in Gestalt von Blasen vorliegen, wobei die Hohlräume mit Luft oder einem Gas, vorzugsweise mit einem inerten Gas, besonders bevorzugt mit Stickstoff, Kohlendioxid, Helium oder einem Gemisch mindestens zweier dieser Gase, gefüllt sind.

13. Mehrschichtiger oraler Dünnfilm gemäß irgendeinem der Ansprüche 11 oder 12, wobei die Hohlräume miteinander in Verbindung stehen und vorzugsweise ein die Matrixschicht durchdringendes Kanalsystem bilden.

14. Mehrschichtiger oraler Dünnfilm gemäß irgendeinem der Ansprüche 11 bis 13, wobei die Hohlräume einen Volumenanteil von 5 bis 98 %, bevorzugt von 50 bis 80 %, bezogen auf das Gesamtvolumen der Matrixschicht, in der Matrixschicht.

15. Mehrschichtiger oraler Dünnfilm gemäß irgendeinem der vorhergehenden Ansprüche, wobei der orale Dünnfilm eine raue Seite mit einer mittleren Rauheit Ra größer 2,0 µm und eine glatte Seite mit einer mittleren Rauheit Ra kleiner als 1,0 µm besitzt.

16. Mehrschichtiger oraler Dünnfilm gemäß irgendeinem der vorhergehenden Ansprüche, wobei der orale Dünnfilm eine raue Seite und eine glatte Seite besitzt, wobei die raue Seite eine mittleren Rauheit Ra von 1,0 µm mehr als die glatte Seite besitzt.

17. Mehrschichtiger oraler Dünnfilm gemäß irgendeinem der vorhergehenden Ansprüche, wobei der mehrschichtige orale Dünnfilm eine mindestens 30% langsamere Freisetzung des mindestens einen pharmazeutisch aktiven Wirkstoffs aufweist, wie die Matrixschicht alleine ohne Rückschicht.

18. Verfahren zur Herstellung eines mehrschichtigen oralen Dünnfilm gemäß irgendeinem der Ansprüche 1 bis 17, umfassend die Schritte
a) Herstellen und Ausstreichen einer Lösung oder Suspension, umfassend das mindestens eine Polyethylenglycol und anschließendes Trocknen der ausgestrichenen Lösung oder Suspension, um einen Film, umfassend das mindestens eine Polyethylenglycol zu erhalten,
b) Herstellen einer Lösung, Dispersion oder Schmelze, die mindestens das mindestens eine Polymer und den mindestens einen pharmazeutischen Wirkstoff umfasst,
b1) optional Aufschäumen der Lösung, Dispersion oder Schmelze aus Schritt b) durch Eintragen eines Gases oder Gasgemisches, durch chemische Gaserzeugung oder durch Entspannen eines gelösten Gases,
c) Ausstreichen der Lösung, Dispersion oder Schmelze aus Schritt b) bzw. der optional aufgeschäumten Lösung, Dispersion oder Schmelze aus Schritt b1) auf den in Schritt a) erhaltenen Film, umfassend das mindestens eine Polyethylenglycol, um einen Verbund zu erhalten,
d) Trocknen des in Schritt c) erhaltenen Verbundes, um einen mehrschichtigen oralen Dünnfilm zu erhalten.

19. Verfahren gemäß Anspruch 18, dadruch gekennzeichnet, dass die Schritte c) und d) durch die folgenden Schritte:
c2) Ausstreichen der Lösung, Dispersion oder Schmelze aus Schritt b) bzw. der optional aufgeschäumten Lösung, Dispersion oder Schmelze aus Schritt b1), um einen Film, umfassend das mindestens eine Polymer und den mindestens einen pharmazeutischen Wirkstoff zu erhalten
d2) Verbinden der in Schritt a) und c2) erhaltenen Filme, vorzugsweise durch Laminieren mittels Wärmeeinwirkung über den Schmelzpunkt eines der in den Filmen enthaltenen Polymere, um einen mehrschichtigen oralen Dünnfilm zu erhalten,
ersetzt werden.

20. Mehrschichtiger oraler Dünnfilm gemäß irgendeinem der Ansprüche 1 bis 17 oder eines mehrschichtigen oralen Dünnfilms erhältlich gemäß dem Verfahren nach Anspruch 18 oder 19 zur Verwendung als Arzneimittel.

## Claims

1. A multi-layer oral thin film comprising a matrix layer, which contains at least one polymer and at least one pharmaceutically active agent, and at least one backing layer, wherein the at least one backing layer comprises at least one polyethylene glycol in an amount of from 60 to 100 wt.%, in relation to the total weight of the at least one backing layer.

2. The multi-layer oral thin film according to claim 1, wherein the matrix layer comprises at least one water-soluble polymer.

3. The multi-layer oral thin film according to claim 2, wherein the at least one water-soluble polymer is selected from the group comprising starch and starch derivatives, dextrans, cellulose derivatives, such as carboxymethyl cellulose, hydroxypropyl cellulose, hydroxyethyl cellulose, hydroxypropyl methylcellulose, hydroxypropyl ethyl cellulose, sodium carboxymethyl cellulose, ethyl or propyl cellulose, polyacrylic acids, polyacrylates, polyvinylpyrrolidones, vinyl pyrrolidone/vinyl acetate copolymers, polyvinyl alcohols, polyethylene oxide polymers, polyacrylamides, polyethylene glycols, gelatines, collagen, alginates, pectin, pullulan, tragacanth, chitosan, alginic acid, arabinogalactan, galactomannan, agar, agarose, carrageenan, and natural gums.

4. The multi-layer oral thin film according to any one of the preceding claims, wherein the at least one pharmaceutically active agent is selected from the group comprising the active agent classes of analgesics, hormones, hypnotics, sedatives, antiepiletics, analeptics, psychoneurotropic drugs, neuro-muscle blockers, antspasmodics, antihistamines, antiallergics, cardiotonics, antiarrhythmics, diuretics, hypotensives, vasopressors, antidepressants, antitussives, expectorants, thyroid hormones, sexual hormones, antidiabetics, antitumour active agents, antibiotics, chemotherapeutics and narcotics, wherein the at least one pharmaceutically active agent preferably comprises ketamine, especially preferably (S)-ketamine.

5. The multi-layer oral thin film according to any one of the preceding claims, wherein the matrix layer further comprises at least one auxiliary substance selected from the group comprising colouring agents, flavourings, sweeteners, plasticisers, taste-masking agents, emulsifiers, enhancers, pH regulators, humectants, preservatives and/or antioxidants.

6. The multi-layer oral thin film according to any one of the preceding claims, wherein the at least one polyethylene glycol has a mean molecular weight of from 20,000 g/mol to 7,000,000 g/mol, preferably from 40,000 g/mol to 500,000 g/mol, especially preferably from 95,000 g/mol to 105,000 g/mol, especially of about 100,000 g/mol.

7. The multi-layer oral thin film according to any one of the preceding claims, wherein the at least one polyethylene glycol has a viscosity of from 30 mPa s to 50 mPa s, measured in 5 wt.% aqueous solution at 25°C.

8. The multi-layer oral thin film according to any one of the preceding claims, wherein the at least one polyethylene glycol is contained in the at least one backing layer in an amount of from 80 to 100 wt.%, in relation to the total weight of the at least one backing layer.

9. The multi-layer oral thin film according to any one of the preceding claims, wherein the at least one backing layer contains at least one plasticiser, preferably glycerol, preferably in an amount of from 0.5 to 5 wt.% in relation to the total weight of the at least one backing layer.

10. The multi-layer oral thin film according to any one of the preceding claims, wherein the multi-layer oral thin film consists of exactly two layers, specifically the matrix layer containing at least one polymer and at least one pharmaceutically active agent and the backing layer containing at least one polyethylene glycol.

11. The multi-layer oral thin film according to any one of the preceding claims, wherein the matrix layer is present in the form of a solidified foam that has voids.

12. The multi-layer oral thin film according to claim 11, wherein the voids are isolated from one another and are preferably present in the form of bubbles, wherein the voids are filled with air or a gas, preferably with an inert gas, especially preferably with nitrogen, carbon dioxide, helium or a mixture of at least two of these gases.

13. The multi-layer oral thin film according to any one of claims 11 or 12, wherein the voids are connected to one another and preferably form a channel system penetrating the matrix layer.

14. The multi-layer oral thin film according to any one of claims 11 to 13, wherein the voids in the matrix layer account for a volume fraction of from 5 to 98%, preferably from 50 to 80%, in relation to the total volume of the layer in question.

15. The multi-layer oral thin film according to any one of the preceding claims, wherein the oral thin film has a rough side with an average roughness Ra greater than 2.0 µm and a smooth side with an average roughness Ra less than 1.0 µm.

16. The multi-layer oral thin film according to any one of the preceding claims, wherein the oral thin film has a rough side and a smooth side, wherein the rough side has an average roughness Ra of 1.0 µm more than the smooth side.

17. The multi-layer oral thin film according to any one of the preceding claims, wherein the multi-layer oral thin film has an at least 30% slower release of the at least one pharmaceutically active agent than the matrix layer alone without backing layer.

18. A method for producing a multi-layer oral thin film according to any one of claims 1 to 17, comprising the steps of:
a) producing and spreading a solution or suspension comprising the at least one polyethylene glycol, and then drying the spread solution or suspension in order to obtain a film comprising the at least one polyethylene glycol,
b) producing a solution, dispersion or melt which at least comprises the at least one polymer and the at least one pharmaceutical active agent,
b1) optionally foaming the solution, dispersion or melt from step b) by introducing a gas or gas mixture by chemical gas generation or by expansion of a dissolved gas,
c) spreading the solution, dispersion or melt from step b) or the optionally foamed solution, dispersion or melt from step b1) onto the film obtained in step a) comprising the at least one polyethylene glycol in order to obtain a composite,
d) drying the composite obtained in step c) in order to obtain a multi-layer oral thin film.

19. The method according to claim 18, **characterised in that** steps c) and d) are replaced by the following steps:
c2) spreading the solution, dispersion or melt from step b) or the optionally foamed solution, dispersion or melt from step b1) in order to obtain a film comprising the at least one polymer and the at least one pharmaceutical active agent,
d2) connecting the films obtained in step a) and c2), preferably by lamination by exposure to heat above the melting point of one of the polymers contained in the films in order to obtain a multi-layer oral thin film.

20. Multi-layer oral thin film according to any one of claims 1 to 17 or of a multi-layer oral thin film obtained by the method according to claim 18 or 19 for the use as a medicament.

## Revendications

1. Film mince oral multicouche, comprenant une couche de matrice contenant au moins un polymère et au moins un principe actif pharmaceutique et au moins une couche arrière, dans lequel l'au moins une couche arrière comprend au moins un polyéthylène glycol en une quantité de 60 à 100 % en poids, par rapport au poids total de l'au moins une couche arrière.

2. Film mince oral multicouche selon la revendication 1, dans lequel la couche de matrice comprend au moins un polymère soluble dans l'eau.

3. Film mince oral multicouche selon la revendication 2, dans lequel l'au moins un polymère soluble dans l'eau est choisi dans le groupe comprenant l'amidon et les dérivés d'amidon, les dextranes, les dérivés de cellulose, tels que la carboxyméthylcellulose, l'hydroxypropylcellulose, l'hydroxyéthylcellulose, l'hydroxypropylméthylcellulose, l'hydroxypropyléthylcellulose, la carboxyméthylcellulose de sodium, l'éthylcellulose ou la propylcellulose, l'acide polyacrylique, les polyacrylates, la polyvinylpyrrolidone, les copolymères de vinylpyrrolidone/acétate de vinyle, les alcools polyvinyliques, les polymères d'oxyde de polyéthylène, les polyacrylamides, les polyéthylènes glycol, la gélatine, le collagène, les alginates, la pectine, le pullulane, la gomme adragante, le chitosan, l'acide alginique, l'arabinogalactane, le galactomannane, la gélose, l'agarose, le carraghénane et les gommes naturelles.

4. Film mince oral multicouche selon l'une quelconque des revendications précédentes, dans lequel l'au moins un principe actif pharmaceutique est de préférence choisi dans le groupe comprenant les classes de principes actifs des analgésiques, des hormones, des hypnotiques, des sédatifs, des antiépileptiques, des weckamines, des psychoneurotropes, des bloqueurs neuromusculaires, des antispasmodiques, des antihistaminiques, des antiallergiques, des cardiotoniques, des antiarythmiques, des diurétiques, des hypotenseurs, des vasopresseurs, des antidépresseurs, des antitussifs, des expectorants, des hormones thyroïdiennes, des hormones sexuelles, des antidiabétiques, des principes actifs antitumoraux, des antibiotiques, des chimiothérapeutiques et des narcotiques, dans lequel l'au moins un principe actif pharmaceutique comprend de préférence la kétamine, de manière particulièrement préférée la (S)-kétamine.

5. Film mince oral multicouche selon l'une quelconque des revendications précédentes, dans lequel la couche de matrice comporte en outre au moins un excipient choisi dans le groupe comprenant les colorants, les arômes, les édulcorants, les plastifiants, les agents de masquage de goût, les émulsifiants, les exhausteurs, les régulateurs de pH, les humectants, les conservateurs et/ou les antioxydants.

6. Film mince oral multicouche selon l'une quelconque des revendications précédentes, dans lequel l'au moins un polyéthylène glycol présente un poids moléculaire moyen de 20 000 g/mol à 700 000 g/mol, de préférence de 40 000 g/mol à 500 000 g/mol, de manière particulièrement préférée de 95 000 g/mol à 105 000 g/mol, en particulier d'environ 100 000 g/mol.

7. Film mince oral multicouche selon l'une quelconque des revendications précédentes, dans lequel l'au moins un polyéthylène glycol présente une viscosité de 30 mPa s à 50 mPa s, mesurée dans une solution aqueuse à 5 % en poids à 25 °C.

8. Film mince oral multicouche selon l'une quelconque des revendications précédentes, dans lequel l'au moins un polyéthylène glycol est contenu en une quantité de 80 à 100 % en poids, par rapport au poids total de l'au moins une couche arrière, dans l'au moins une couche arrière.

9. Film mince oral multicouche selon l'une quelconque des revendications précédentes, dans lequel l'au moins une couche arrière contient au moins un plastifiant, de préférence du glycérol, de préférence en une quantité de 0,5 à 5 % en poids, par rapport au poids total de l'au moins une couche arrière.

10. Film mince oral multicouche selon l'une quelconque des revendications précédentes, dans lequel le film mince oral multicouche est constitué d'exactement deux couches, à savoir la couche de matrice contenant au moins un polymère et au moins un principe actif pharmaceutique et la couche arrière contenant au moins un polyéthylène glycol.

11. Film mince oral multicouche selon l'une quelconque des revendications précédentes, dans lequel la couche de matrice est présente sous forme d'une mousse solidifiée présentant des cavités.

12. Film mince oral multicouche selon la revendication 11, dans lequel les cavités sont isolées les unes des autres et se présentent de préférence sous la forme de bulles, dans lequel les cavités sont remplies d'air ou d'un gaz, de préférence d'un gaz inerte, de manière particulièrement préférée d'azote, de dioxyde de carbone, d'hélium ou d'un mélange d'au moins deux de ces **gaz.**

13. Film mince oral multicouche selon l'une quelconque des revendications 11 ou 12, dans lequel les cavités sont reliées entre elles et forment de préférence un système de canaux pénétrant la couche de matrice.

14. Film mince oral multicouche selon l'une quelconque des revendications 11 à 13, dans lequel les cavités présentent un pourcentage volumique de 5 à 98 %, de préférence de 50 à 80 %, par rapport au volume total de la couche de matrice, dans la couche de matrice.

15. Film mince oral multicouche selon l'une quelconque des revendications précédentes, dans lequel le film mince oral possède un côté rugueux avec une rugosité moyenne Ra supérieure à 2,0 µm et un côté lisse avec une rugosité moyenne Ra inférieure à 1,0 µm.

16. Film mince oral multicouche selon l'une quelconque des revendications précédentes, dans lequel le film mince oral possède un côté rugueux et un côté lisse, dans lequel le côté rugueux possède une rugosité moyenne Ra de 1,0 µm de plus que le côté lisse.

17. Film mince oral multicouche selon l'une quelconque des revendications précédentes, dans lequel le film mince oral multicouche présente une libération de l'au moins un principe actif pharmaceutique au moins 30 % plus lente que la couche de matrice seule sans couche arrière.

18. Procédé pour la fabrication d'un film mince oral multicouche selon l'une quelconque des revendications 1 à 17, comprenant les étapes
a) de préparation et d'étalement d'une solution ou suspension, comprenant l'au moins un polyéthylène glycol, puis de séchage de la solution ou suspension étalée pour obtenir un film comprenant l'au moins un polyéthylène glycol,
b) de préparation d'une solution, dispersion ou masse fondue, qui comprend au moins l'au moins un polymère et l'au moins un principe actif pharmaceutique,
b1) éventuellement de moussage de la solution, dispersion ou masse fondue de l'étape b) par introduction d'un gaz ou mélange de gaz, par production chimique de gaz ou par détente d'un gaz dissous,
c) d'étalement de la solution, dispersion ou masse fondue de l'étape b) ou de la solution, dispersion ou masse fondue éventuellement moussée de l'étape b1) sur le film obtenu à l'étape a), comprenant l'au moins un polyéthylène glycol, pour obtenir un ensemble,
d) de séchage de l'ensemble obtenu à l'étape c) pour obtenir un film mince oral multicouche.

19. Procédé selon la revendication 18, **caractérisé en ce que** les étapes c) et d) sont remplacées par les étapes suivantes :
c2) l'étalement de la solution, dispersion ou masse fondue de l'étape b) ou de la solution, dispersion ou masse fondue éventuellement moussée de l'étape b1) pour obtenir un film comprenant l'au moins un polymère et l'au moins un principe actif pharmaceutique
d2) la liaison des films obtenus à l'étape a) et c2), de préférence par lamination par action thermique au-dessus du point de fusion d'un des polymères contenus dans les films pour obtenir un film mince oral
multicouche.

20. Film mince oral multicouche selon l'une quelconque des revendications 1 à 17 ou d'un film mince oral multicouche pouvant être obtenu selon le procédé selon la revendication 18 ou 19 destiné à être utilisé comme médicament.
